# EUROPEAN PATENT SPECIFICATION

(11) **EP 2 823 094 B1**
(45) Date of publication and mention of the grant of the patent: **06.10.2021**
(21) Application number: 13758480.1
(22) Date of filing: 08.03.2013
(51) Int. Cl.: D01F 11/14, C07D 207/06, C07D 403/12, C09C 1/44, B82Y 30/00, B82Y 40/00, C07D 487/04, C01B 32/16, C01B 32/168, C01B 32/174

(54) **COVALENT FUNCTIONALIZATION OF CARBON NANOTUBES GROWN ON A SURFACE**
KOVALENTE FUNKTIONALISIERUNG VON AUF EINER OBERFLÄCHE GEZÜCHTETEN KOHLENSTOFFNANORÖHRCHEN
FONCTIONNALISATION COVALENTE DE NANOTUBES DE CARBONE AMENÉS À CROÎTRE SUR UNE SURFACE

(30) Priority: 09.03.2012 US 201261609011 P
(43) Date of publication of application: 14.01.2015
(73) Proprietor: Johansson, Johan, 413 09 Gothenburg (SE); Khalid, Waqas, Berkeley, California 94704 (US)
(72) Inventor: KHALID, Waqas, Berkeley, California 94704 (US); JOHANSSON, Johan, SE-413 09 Gothenburg (SE)
(74) Representative: Adam, Holger
(86) International application number: PCT/IB2013/000954
(87) International publication number: WO 2013/132352

(56) References cited:
- WO-A1-2004/089819
- US-A1- 2005 269 285
- US-A1- 2007 134 866
- SIMONE M. KANIBER ET AL: "On-Chip Functionalization of Carbon Nanotubes with Photosystem I", JOURNAL OF THE AMERICAN CHEMICAL SOCIETY, vol. 132, no. 9, 10 March 2010 (2010-03-10), pages 2872-2873, XP055161899, ISSN: 0002-7863, DOI: 10.1021/ja910790x
- ALBERTO BIANCO ET AL: "Cationic Carbon Nanotubes Bind to CpG Oligodeoxynucleotides and Enhance Their Immunostimulatory Properties", JOURNAL OF THE AMERICAN CHEMICAL SOCIETY, vol. 127, no. 1, 16 December 2004 (2004-12-16), pages 58-59, XP055217125, ISSN: 0002-7863, DOI: 10.1021/ja044293y
- Alberto Bianco ET AL: "Carbon Nanotube-based Vectors for Delivering Immunotherapeutics and Drugs", Nanotechnologies for the Life Sciences, 15 September 2007 (2007-09-15), pages 85-142, XP055208076, Retrieved from the Internet: URL:http://www.wiley-vch.de/books/sample/3 527313907_c01.pdf [retrieved on 2015-08-17]
- ENRIQUE LALLANA ET AL: "Click Chemistry for Drug Delivery Nanosystems", PHARMACEUTICAL RESEARCH, KLUWER ACADEMIC PUBLISHERS-PLENUM PUBLISHERS, NL, vol. 29, no. 1, 13 September 2011 (2011-09-13), pages 1-34, XP019993267, ISSN: 1573-904X, DOI: 10.1007/S11095-011-0568-5
- SALEH TANVEER ET AL: "Field-emission-assisted approach to dry micro-electro-discharge machining of carbon-nanotube forests", JOURNAL OF APPLIED PHYSICS, AMERICAN INSTITUTE OF PHYSICS, US, vol. 110, no. 10, 15 November 2011 (2011-11-15), pages 103305-103305, XP012153899, ISSN: 0021-8979, DOI: 10.1063/1.3663438 [retrieved on 2011-11-29]
- A. GARCÍA ET AL: "Synthesis of dendrimer-carbon nanotube conjugates", PHYSICA STATUS SOLIDI (A), vol. 205, no. 6, 1 June 2008 (2008-06-01), pages 1402-1407, XP055207980, ISSN: 1862-6300, DOI: 10.1002/pssa.200778146
- GEORGAKILAS V ET AL: "Organic Functionalization of Carbon Nanotubes", JOURNAL OF THE AMERICAN CHEMICAL SOCIETY, AMERICAN CHEMICAL SOCIETY, US, vol. 124, no. 5, 6 February 2002 (2002-02-06), pages 760-761, XP002262088, ISSN: 0002-7863, DOI: 10.1021/JA016954M
- JOHANSSON ET AL.: 'Covalent functionalization of carbon nanotube forests grown in situ on a metal- silicon chip' NANOSENSORS, BIOSENSORS, AND INFO-TECH SENSORS AND SYSTEMS 2012, PROCEEDINGS OF THE SPIE vol. 8344, 2012, XP055161897
- LEYDEN ET AL.: 'Fabrication and characterization of carbon nanotube field-effect transistor biosensors' ORGANIC SEMICONDUCTORS IN SENSORS AND BIOELECTRONICS III 17 August 2010, pages 77790H-1 - 77790H-11, XP055161898
- KANIBER ET AL.: 'On- chip functionalization of carbon nanotubes with photosystem 1' J. AM. CHEM. SOC. vol. 132, no. 9, 2010, pages 2872 - 2873, XP055161899
- None

## Description

### FIELD OF THE INVENTION

The present invention is related to a new method for directly covalently functionalizing carbon nanotubes (CNTs) grown on or attached to a surface. The invention also features devices that are comprised of CNTs.

### BACKGROUND OF THE INVENTION

Carbon nanotubes (CNTs) possess unique properties such as small size and electrical conductivity, which makes them suitable in a wide range of potential applications, including use as structural materials in molecular electronics, nanoelectronic components, nanosensors and field emission displays. Carbon nanotubes may be either multi-walled (MWNTs) or single-walled (SWNTs), and have diameters in the nanometer range. Depending on their atomic structure CNTs may have either metallic or semiconductor properties, and in combination with their small dimensions these properties make them particularly attractive for use in fabrication of nanodevices.

Carbon nanotube forests have been the focus of interest for research in various areas of science and technology including in the context of design of microelectronics, super capacitors and biosensors. The unique electrical, mechanical, optical and thermal properties of CNT forests are key factors that make such CNT structures interesting candidate for numerous nano technology applications. Various efforts to functionalize CNTs chemically have been reported in literature where the CNTs are functionalized by covalent and non-covalent attachment of functional groups. The conventional methods usually employ the technique to functionalize the CNTs when dispersed in solution. After the functionalization reactions, the CNTs are deposited on a surface. Generally the effort to functionalize the CNTs is carried out to enhance the solubility of the CNTs in various solvents. Moreover, harsh oxidizing agents like nitric acid or strong bases are frequently used. Such techniques cannot be employed to functionalize CNTs when on chip, as the underlying metal films are attacked by the reagents. Moreover, the dispersive technique leads to larger process errors and issues with reproducibility of devices.

Alberto Bianco ET AL: "Carbon Nanotube-based Vectors for Delivering Immunotherapeutics and Drugs", Nanotechnologies for the Life Sciences, 15 September 2007 (2007-09-15) and Alberto Bianco ET AL: "Cationic Carbon Nanotubes Bind to CpG Oligodeoxynucleotides and Enhance Their Immunostimulatory Properties", Journal of the American chemical society, 16 December 2004, vol. 127, no. 1 disclose ways to make the CNTs soluble in solvents and then functionalize them to re-attach them to a surface of a sensor chip.

Accordingly, there is a need in the art to find a method to functionalize CNT structures as grown on chip. This reduces the fabrication errors in reproducibility of devices with almost the same properties. Moreover, the use of such structures as sensors is much simpler, economical and CMOS compatible when they are functionalized as grown on chips using reactions at relatively low temperatures and atmospheric pressure, and without any aggressive chemicals. Further, the underlying metal films that are used to ensure good electrical contact between all the CNTs in a CNT forest are prevented from any damage. The combination of the electrical properties of carbon nanotube forests, their morphology and the ease of functionalization method provides novel ways of immobilizing biomolecules on extremely large surface areas of these structures, providing a potential platform for making ultra-sensitive biosensors.

### SUMMARY OF THE INVENTION

It is an object of the present invention to provide methods for directly covalently functionalizing carbon nanotubes attached to or assembled by growth on a surface. The methods of the present invention can be employed to functionalized CNT structures regardless of the requirement that the nanostructures are grown on an electrical portion on a substrate. Such a technique is simpler and is more convenient to functionalize nanostructures without the need for electrical portions attachments. Thus, it reduces/ eliminates the risk of short circuiting (in comparison to the functionalization of the CNT structures with electrochemical functionalization) and the simpler setup for functionalization of nanostructures with mild conditions makes it a process that is compatible with CMOS and electronics.

Thus, in one embodiment, the present invention features a method of adding a chemical attachment to the nanotubes A that can then have a linker L and that in turn hosts the functional groups (FG)n. The first chemical modification to the CNT nanostructures can change the hydrophobicity of the structures (*i.e*. making them hydrophobic or hydrophilic) or it can be a linker for the attachment of a catalyst or a fluorescent tag or a biomarker or biomolecules.

It is another object of the present invention to provide the base of a platform that can be used in a broad range of applications, ranging from electrical components, small smart detectors for even a single molecule detection, super capacitors, solar cells, gas storage, imaging devices, biosensors, nano tweezers for molecular manipulation, cell manipulation, an interface for molecular electronics, in microfluidics or as foundation of immobilized catalysts for organic synthesis and inorganic synthesis or as stationary phase in compound purification systems. The carbon nanotube structures do not necessarily have to be attached to a conducting portion on a substrate for the chemical reaction to take place. Thus, individual addressability electrically is not needed for the reactions to take place.

Accordingly, in a first aspect, the present disclosure provides a method of functionalizing a carbon nanotube (CNT) on a surface of a chip according to formula I:

SURFACE- CNT-A-L-(FG)n, I

wherein A is the attachment to the CNT, L is a linker between the attachment on the CNT and one or more functional group (FG)n. In further related embodiments, the linker L can be a functional group.

In one embodiment, the method further comprises a cycloaddition step, an amide coupling step, and a CuAAC step.

In another embodiment, the functional group is covalently attached.

In another aspect, the disclosure features a method of covalently functionalizing carbon nanotubes (CNTs) grown on a metal film surface according to formula I SURFACE-CNT-A-L-(FG)n, comprising a cycloaddition step, an amide coupling step, and a copper(I)-catalyzed azide alkyne cycloaddition CuAAC step,
wherein A is the attachment to the CNTs, L is a linker between the attachment on the CNTs and the one or more functional groups (FG)n. In further related embodiments, the linker L or A can be a functional group.

In one embodiment, A is a pyrrolidine ring substituted with L on either the nitrogen atom or on C2 or C5 of the pyrrolidine ring system.

In another embodiment, L is substituted with R1 and R2.

In another embodiment, A is substituted with R1, R2 and L.

In another further embodiment, the C3 and C4 in A originate from the CNT structure.

In still another embodiment, R1 and R2 are each and independently selected from hydrogen, (C₁-C₁₅)alkyl, (C₃-C₈)cycloalkyl, aryl, heterocyclyl, or any of these groups (except hydrogen) optionally substituted with one or more halogen (F, Cl, Br or I) atoms and/or one or more of the following groups, OH, CN, NO₂, (C₁-C₁₂)alkyl, (C₁-C₁₂)alkoxyC(O), halogen substituted (C₁-C₁₂)alkyl, (C₃-C₆)cycloalkyl, aryl, hetrocyclyl, optionally interrupted by one or more, O, N, S, P, Si or B atoms.In another embodiment, L is a linker between the attachment A on the CNTs and the FG, selected from the group consisting of: (C₁-C₃₀)alkyl, (C₃-C₈)cycloalkyl, aryl, heterocyclyl, and any of these groups (except hydrogen) optionally interrupted by one or more O, N, S, P, Si, or B atoms, and/or one or more carbonyls, and/or substituted by one or more halogen (F, Cl, Br or I) atoms and/or one or more of the following groups, (C₁-C₁₂)alkyl, (C₁-C₁₂)alkoxyC(O), halogen substituted (C₁-C₁₂)alkyl, (C₃-C₆)cycloalkyl, aryl, hetrocyclyl, optionally interrupted by one or more, O, N, S, P, Si or B atoms.

In another embodiment, L contains one or more carbon atoms.

In another further embodiment, the L is bound to a nitrogen or C2 or C5 on the pyrrolidine ring system.

In another embodiment, the cycoladdition reaction is a 1,3-dipolar cycloaddition.

In another further embodiment, the FG is selected from the group consisting of: amine, azide, alkyne, carboxylic acid, an activated ester of a carboxylic acid, thiol, acid chloride, acid fluoride, sulfonyl chloride, isocyanate, isothiocyanate, aryl halide (F, Cl, Br or I), alkyl halide (F, Cl, Br or I), maleimide, ketone, aldehyde, alcohol, alkene, triflate, nitro, nitrile, boronic acid, boronic ester and a trifluoroborate salt.

In still another embodiment, the method is performed in a solvent selected from the group consisting of: toluene, benzene, chlorobenzene, dichlorobenzene, poly halogenated benzene derivatives, trifluoro methylbenzene, CH₂Cl₂, CHCl₃, CCl₄, DCE, DMF, DMA, NMP, dioxane, THF, 2-MeTHF and DMSO.

In another aspect, the present invention features a composition comprising a carbon nanotube (CNT) on a surface of a chip according to formula I:

SURFACE- CNT-A-L-(FG)n, I

wherein A is the attachment to the CNT, L is a linker between the attachment on the CNT and one or more functional group (FG)n.

In one embodiment, the functional group is covalently attached.

In another embodiment, the surface is a metal film.

In another further embodiment, A is a pyrrolidine ring substituted with L on either the nitrogen atom or on C2 or C5 of the pyrrolidine ring system.

In another embodiment, L is a linker between the attachment A on the CNTs and the (FG)n, selected from the group consisting of: (C₁-C₃₀)alkyl, (C₃-C₈)cycloalkyl, aryl, heterocyclyl, and any of these groups (except hydrogen) optionally interrupted by one or more O, N, S, P, Si, or B atoms, and/or one or more carbonyls, and/or substituted by one or more halogen (F, Cl, Br or I) atoms and/or one or more of the following groups, (C₁-C₁₂)alkyl, (C1-C₁₂)alkoxyC(O), halogen substituted (C₁-C₁₂)alkyl, (C₃-C₆)cycloalkyl, aryl, hetrocyclyl, optionally interrupted by one or more, O, N, S, P, Si or B atoms.

In another further embodiment, L contains one or more carbon atoms.

In another embodiment, the L is bound to the nitrogen or C2 or C5 on the pyrrolidine ring system.

In another embodiment, the FG is selected from the group consisting of: amine, azide, alkyne, carboxylic acid, an activated ester of a carboxylic acid, thiol, acid chloride, acid fluoride, sulfonyl chloride, isocyanate, isothiocyanate, aryl halide (F, Cl, Br or I), alkyl halide (F, Cl, Br or I), maleimide, ketone, aldehyde, alcohol, alkene, triflate, nitro, nitrile, boronic acid, boronic ester and a trifluoroborate salt.

In another aspect, the present invention features a device comprising one or a plurality of CNT structures as described herein.

In one embodiment, the CNT structures (with a high surface area 3D set up) can be functionalized in solid, liquid, gaseous or liquid crystal or any other phases.

In another embodiment, there is energy exchange, transfer or storage between the CNT structures and the functionalizations or between the plurality of CNT structures that are functionalized.

In another embodiment, there is energy exchange, the plurality of CNT structures are covalently functionalized on-chip.

### BRIEF DESCRIPTION OF THE DRAWINGS

Referring to the drawings in general, it will be understood that the illustrations are for the purpose of describing particular embodiments of the disclosure and are not intended to be limited thereto.
Figure 1 shows a schematic of the fabrication process of CNT structures.
Figure 2 shows a picture and a schematic drawing of the CNT forests on chip. The black squares in the picture (left) are CNT forests and the schematic drawing (middle) shows how the CNT forests are bound to the surface on chip. A SEM micrograph of CNT forest consists of a bundle of many vertically aligned CNTs (right).
Figure 3 shows a schematic reaction scheme of the functionalization process for one example of the invention. Reaction conditions: (a) Glycine, benzaldehyde, dichlorobenzene, 120 °C for 6 to 48 h. (b) PyBOP, O-(2-azidoethyl)-O-[2-(diglycolyl-amino)ethyl]heptaethylene glycol, DIPEA, CH2Cl2, 24 h at room temperature (r.t.) (c) 1-ethynyl-4-(trifluoromethyl)benzene, CuSO4, sodium ascorbate, DMA/water 3:1, 40 h at r.t.
Figure 4 (a and b) (a) shows the reaction setup (after reaction) and (b) shows the cleaning setup, done for at least 1 h three times after any reaction step.
Figure 5 (a and b) is two panels (a) shows a SEM micrograph of CNT forests grown on chip. (b) shows the EDS spectrum of non functionalized CNT structures.
Figure 6 (a and b) (a) shows SEM micrograph of f-CNT 1, with an enlargement of the section where EDS was performed.. (b) The EDS spectrum of the f-CNT 1 showing a small nitrogen peak at 0.39 keV.
Figure 7 (a and b) (a) shows a SEM micrograph of the section of CNT forest where the EDS was performed on f-CNT 2 (b) the EDS spectrum of f-CNT 2 shows a clear nitrogen peak at 0.39 keV.
Figure 8 shows the SEM micrograph and the EDS spectrum of f-CNT 3.
Figure 9 shows a SEM micrograph of a section with no CNT forests, where the EDS was performed on f-CNT 3.
Figure 10 shows a SEM micrograph of the section of CNT forest where EDS was performed on the control chip.

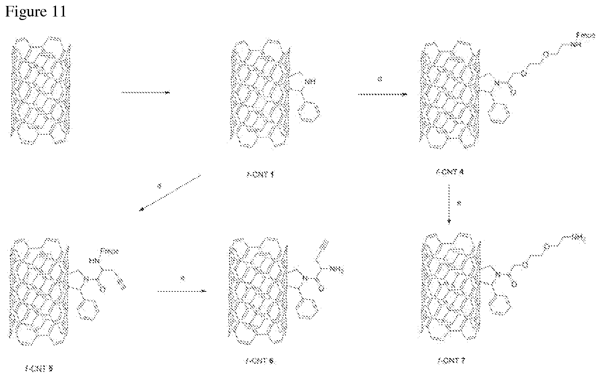

Figure 11 shows a schematic reaction scheme for other examples of the invention. Reaction conditions: (d) PyBop, DIPEA, carboxylic acid, ambient temperature 24 h, (e) 20% piperidine in DMF, ambient temperature 24 h.

### DETAILED DESCRIPTION

Unless otherwise noted, technical terms are used according to conventional usage. As utilized in accordance with the present disclosure, the following terms shall be understood to have the following meanings:
The term "carbon nanotube (CNT)" is meant herein to refer to hollow structures composed primarily of carbon atoms. Carbon nano-structures of the present invention are those structures comprised at primarily of carbon which take the form of tubes, rods, cylinders, bundles, wafers, disks, sheets, plates, planes, cones, slivers, granules, ellipsoids, wedges, polymeric fibers, natural fibers, and other such objects which have at least one characteristic dimension less than about 100 nm. Preferred carbon nanostructures of the invention are nanotubes.

The term "single wall carbon nanotubes" (SWCNT) is meant to refer to carbon nanotubes having one wall.

The term "multi-wall carbon nanotubes" (MWCNT) is meant to refer to carbon nanotubes having more than one wall. In certain embodiments, according to the present definition of multi-wall carbon nanotubes, double- and triple-wall carbon nanotubes will be considered to be multi-wall carbon nanotubes.

The term "carbon nanotube (CNT) chip" is meant to refer to carbon nanotubes grown on a silicon chip.

The term "surface" is meant to refer to a material to which a carbon nanotube may be affixed either by direct chemical means or via an intermediate material such as a coating. An example of a surface is a solid surface.

The term "catalyst" is meant to refer to a substance capable of catalyzing the formation of carbon nanotubes.

The term "nanostructure" is meant to refer to any structure having a diameter or width in the range of nanometers. Examples of such nanostructure includes, but not limited to, carbon nanotubes, nanowires and nanorods of various materials such as for example ZnO, 30 and carbon nano fibers.

The term "set of nanostructures" is meant to refer to a plurality of nanostructures arranged in close proximity (for example from micrometer to submicrometer) to each other on a portion of the substrate, which portion is separated from other portions of the substrate comprising nanostructures, and which portion comprises an electrical portion underlying the set of nanostructure. A set of nanostructures may comprise nanostructures arranged in bundles. A set of nanostructure may correspond to a portion of the substrate having a length and width in the range of from nanometer to micrometer. Further, when a set of nanostructures is being individually addressed all nanostructures comprised in such set are typically addressed.

Carbon nano-structures of the present invention are those structures comprised primarily of carbon which take the form of tubes, rods, cylinders, bundles, wafers, disks, sheets, plates, planes, cones, slivers, granules, ellipsoids, wedges, polymeric fibers, natural fibers, and other such objects which have at least one characteristic dimension less than about 100 nm. Preferred carbon nanostructures of the invention are nanotubes.

CNTs of the invention are generally about 1-200 nm in diameter where the ratio of the length dimension to the narrow dimension, i.e., the aspect ratio, is at least 5. In general, the aspect ratio is between 10 and 2000. Carbon nanotubes are comprised primarily of carbon atoms, however may be doped with other elements, e.g., metals. The carbon-based nanotubes of the invention can be either multi-walled nanotubes (MWNTs) or single-walled nanotubes (SWNTs). A MWNT, for example, includes several concentric nanotubes each having a different diameter. Thus, the smallest diameter tube is encapsulated by a larger diameter tube, which in turn, is encapsulated by another larger diameter nanotube. A SWNT, on the other hand, includes only one nanotube.

The present invention provides covalent functionalizations of well-defined CNTs grown on a surface, of general formula (I) that can be used for various applications as mentioned above. The CNTs growth on a surface is based on previous work that discloses an improved method for manufacturing of a device comprising nanostructures, in particular having individually addressable sets of nanostructures (European Patent Application No. 11171883.9). For example, in certain embodiments, the present invention is based on the realization that sets of nanostructures may be grown on a first substrate comprising an insulating material whilst at the same time providing an electrical portion within the first substrate to electrically connect each individual set of nanostructure, by providing stacks on the first substrate of at least a first and a second layer comprising a first and a second material, respectively, wherein the second layer is arranged on the first layer which in turn is in direct contact with the first substrate. The second material is chosen to catalyze the growth of nanostructure at elevated temperatures, while the first material and the insulating material are chosen such that the first material may, upon heating, mix or diffuse and/or react with the insulating material resulting in an electrically conductive and/or semi-conductive mixture. Thus by heating the first substrate having such stacks arranged thereon, a plurality of sets of nanostructures may be produced wherein the portions of the first substrate underlying the sets of nanostructures comprise a conductive and/or semi-conductive material. Thereby each set of nanostructure may be individually electrically connectable (European Patent Application No. 11171883.9).

For example, in certain embodiments, the present invention features a method for growing set of nanostructures on top of a first substrate comprising an insulating material and at the same time, forming individually addressable electrical connections to each set of nanostructure within the first substrate.

The invention is not limited to any specific method of growing nanostructures and so the composition of the atmosphere may therefore depend on the type of nanostructures grown (e.g. carbon nanotube, single or multi-walled, carbon nanofibers, ZnO nano wires, etc). Methods such as chemical vapor deposition, plasma enhanced chemical vapor deposition, arc discharge, laser ablation, or any other suitable methods known to the skilled person, may typically be used. Thus, the atmosphere may typically comprise at least one of ethylene, argon, plasma, hydrogen, nitrogen and ammonia (for carbon nanotube growth).

According to certain preferred embodiments, the CNT structures are grown on a buffer layer on a substrate. In related embodiments, the buffer layer can be conducting or non-conducting hosting the catalyst to grow CNTs. For example, in certain preferred embodiments, the substrate can be silicon, silicon dioxide, silicon nitride, quarts, glass, carbides, or metal.

The first step of the covalent modification of CNTs on a surface is a 1,3-dipolar cycloaddition.

Formula 1: **Surface-CNT-A-L-(FG)ₙ**

Wherein;
A is the attachment to the CNTs. A is a pyrrolidine ring substituted with L on either the nitrogen atom or on C2 or C5 of the pyrrolidine ring system. Further, A may be substituted with R1 and R2. In this covalent functionalization, the C3 and C4 in A come/originate from the carbon nanotubes structures.

R₁ and R₂ are each and independently selected from hydrogen, (C₁-C₁₅)alkyl, (C₃-C₈)cycloalkyl, aryl, heterocyclyl, and any of these groups (except hydrogen) optionally substituted with one or more halogen (F, Cl, Br or I) atoms and/or one or more of the following groups, OH, CN, NO₂, (C₁-C₁₂)alkyl, (C₁-C₁₂)alkoxyC(O), halogen substituted (C₁-C₁₂)alkyl, (C3-C6)cycloalkyl, aryl, hetrocyclyl, optionally interrupted by one or more, O, N, S, P, Si or B atoms.

L is linker between the attachment A on the CNTs and the FG(s) and defined as: (C₁-C₃₀)alkyl, (C₃-C₈)cycloalkyl, aryl, heterocyclyl, and any of these groups (except hydrogen) optionally interrupted by one or more O, N, S, P, Si, or B atoms, and/or one or more carbonyls, and/or substituted by one or more halogen (F, Cl, Br or I) atoms and/or one or more of the following groups, (C₁-C₁₂)alkyl, (C₁-C₁₂)alkoxyC(O), halogen substituted (C₁-C₁₂)alkyl, (C₃-C₆)cycloalkyl, aryl, hetrocyclyl, optionally interrupted by one or more, O, N, S, P, Si or B atoms. Further one or more of the carbon atoms in L is substituted by one FG (functional group) independently chosen from each other from the defined FGs above (n = 1, 2, 3...). L is bond to the nitrogen or C2 or C5 on the pyrrolidine ring system.

FG is a functional group preferably selected from but not limited to the following: amine, azide, alkyne, carboxylic acid, an activated ester of a carboxylic acid, thiol, acid chloride, acid fluoride, sulfonyl chloride, isocyanate, isothiocyanate, aryl halide (F, Cl, Br or I), alkyl halide (F, Cl, Br or I), maleimide, ketone, aldehyde, alcohol, alkene, triflate, nitro, nitrile, boronic acid, boronic ester or a trifluoroborate salt.

CNTs may be produced by a variety of methods, and are additionally commercially available. Methods of CNT synthesis include laser vaporization of graphite (A. Thess et al. Science 273, 483 (1996)), arc discharge (C. Journet et al., Nature 388, 756 (1997)) and HiPCo (high pressure carbon monoxide) process (P. Nikolaev et al. Chem. Phys. Lett. 313, 91-97 (1999)). Chemical vapor deposition (CVD) can also be used in producing carbon nanotubes (J. Kong et al. Chem. Phys. Lett. 292, 567-574 (1998); J. Kong et al. Nature 395, 878-879 (1998); A. Cassell et al. J. Phys. Chem. 103, 6484-6492 (1999); H. Dai et al. J. Phys. Chem. 103, 11246-11255 (1999)). Additionally CNT's may be grown via catalytic processes both in solution and on solid substrates (Yan Li, et al., Chem. Mater.; 2001; 13(3); 1008-1014); (N. Franklin and H. Dai Adv. Mater. 12, 890 (2000); A. Cassell et al. J. Am. Chem. Soc. 121, 7975-7976 (1999)).

In one embodiment of the invention, the nanostructures composed of CNTs are grown using a catalyst. In various embodiments, the catalyst layer includes a metal, such as, for example, a low-valent or zero-valent metal such as but not limited to Iron or Mo or Ni. Such metal catalysts for forming carbon nanotubes are well known to those of ordinary skill in the art. The CNTs can be grown using CVD or PECVD process using a carbon source like but not limited to ethylene in mixture with a reducing gas such as, but not limited to, hydrogen and a carrier gas such as, but not limited to, nitrogen or argon, or other gases such as, but not limited to, ammonia and or water vapors. In some embodiments, the metal of the catalyst layer may be, for example, iron, nickel, cobalt, or combinations thereof. Variations in catalyst composition and density may be performed by alloying various metals together. Such alloyed catalysts may not only influence the properties of the carbon nanotubes grown, but also affect the interaction of the insulating layer with the substrate and the catalyst layer and dictate the ease with which it detaches from the substrate.

In certain embodiments, the catalyst layer is discontinuous. In embodiments having a discontinuous catalyst layer, catalyst deposition results in the formation of small catalyst or catalyst precursor particles during the deposition step as a result of the balance between catalyst-surface interfacial interactions relative to intra-particle atomic interactions.

In certain embodiments, the catalyst layer is between about 1 nm and about 5 nm in thickness. In other various embodiments, the catalyst layer is between about 0.5 nm and about 5 nm in thickness. In still other various embodiments, the catalyst layer is between about 0.5 nm and about 3 nm in thickness. In some embodiments, the catalyst layer is between about 2 nm and about 5 nm in thickness. In some embodiments, the catalyst layer is less than about 2 nm in thickness.

In some embodiments, the catalyst layer is deposited by electron beam deposition. In other embodiments, the catalyst layer is deposited by atomic layer deposition. In some embodiments, growing of carbon nanotubes takes place by chemical vapor deposition.

In another embodiment of the invention, the catalyst is deposited on the surface of the buffer layer. The buffer layer can be an insulator, for example a metal oxide or non-metal oxide. In exemplary embodiments, the insulator is selected from, but not limited to, SiO₂, Al₂O₃ or Si₃N₄. In some embodiments, the insulating layer is between about 1 nm and about 100 nm in thickness. In other embodiments, the insulating layer is between about 1 nm and about 50 nm in thickness. In still other embodiments, the insulating layer is between about 5 nm and about 50 nm in thickness. In still other embodiments, the insulating layer is between about 1 nm and about 40 nm in thickness. The buffer layer can also be a metal film like but not limited to Al, Ti, Mo, Ta, Cr, Au, Pt, W.

In another further embodiment of the invention, the buffer layer is on a substrate. The substrate can be, but is not limited to, silicon (Si), glass, quartz, a metal, such as, but not limited to, aluminum or an alloy, such as, but not limited to, steel, or a compound, such as, but not limited to, silicon carbide etc.

In another embodiment of the invention, the CNT structures grown on the substrates are covalently functionalized on-chip. According to preferred exemplary embodiments, covalent functionalization of the CNTs is defined by chemical addition of attachment "A" to the CNTs as defined above.

In another embodiment of the invention, the R1 and R2 is a hydrogen, aryl, C₁-C₈ alkyl, hetro cyclyl, and any of these groups (except hydrogen) optionally substituted with one or more halogen (F, Cl, Br or I) atoms and/or one or more of the following groups, OH, CN, NO₂, (C₁-C₄) alkyl.

In another embodiment, R1 is an aryl or hydrogen and R2 is hydrogen.

### Linker (L)

As described herein, according to certain embodiments of the present invention, L is linker between the attachment A on the CNTs and the (FG)n and defined as: (C₁-C₂₂)alkyl, (C₃-C₈)cycloalkyl, aryl, heterocyclyl, and any of these groups (except hydrogen) optionally interrupted by one or more O, N, S atoms, and/or one or more carbonyls, and/or substituted by one or more halogen (F, Cl, Br or I) atoms and/or one or more of the following groups, (C₁-C₆) alkyl, (C₁-C₆) alkoxy C(O), halogen substituted (C₁-C₁₂)alkyl, (C₃-C₆)cycloalkyl, aryl, hetrocyclyl, optionally interrupted by one or more, O, N, S atoms. Further one or more of the carbon atoms in L is substituted by one FG (functional group) independently chosen from each other from the defined FGs above. L is bond to the nitrogen or C2 or C5 on the pyrrolidine ring system.

In another embodiment, L is linker between the attachment A on the CNTs and the FG(s) and defined as: (C₁-C₂₂)alkyl, (C₃-C₈)cycloalkyl, aryl, heterocyclyl, and any of these groups (except hydrogen) optionally interrupted by one or more O, N, S atoms, and/or one or more carbonyls, and/or substituted by one or more halogen (F) atoms and/or one or more of the following groups, (C₁-C₆) alkyl, halogen substituted (C₁-C₄)alkyl, aryl, hetrocyclyl, optionally interrupted by one or more, O, N, S atoms. Further one or more of the carbon atoms in L is substituted by one FG (functional group) independently chosen from each other from the defined FGs above. L is bond to the nitrogen or C2 or C5 on the pyrrolidine ring system.

### Functional Group (FG)

As described herein, according to certain embodiments of the present invention, FG is a functional group preferably selected from the following: amine, azide, alkyne, carboxylic acid, an activated ester of a carboxylic acid, thiol, isocyanate, isothiocyanate, aryl halide (F, Cl, Br or I), alkyl halide (Cl, Br or I), maleimide, ketone, aldehyde, alcohol, alkene, nitro, nitrile, boronic acid, boronic ester or a trifluoroborate salt.

In another embodiment of the invention, FG is a functional group preferably selected from the following: amine, azide, alkyne, carboxylic acid, an activated ester of a carboxylic acid, thiol, isocyanate, isothiocyanate, alkyl halide (Cl, Br or I), maleimide, ketone, aldehyde, alcohol, alkene, nitro.

In another embodiment of the invention, FG is a functional group preferably selected from the following: amine, azide, alkyne, carboxylic acid, an activated ester of a carboxylic acid, isothiocyanate, alkyl halide (Cl, Br or I).

In further embodiment of the invention the FG is selected from: azide, alkyne, amine, or carboxylic acid.

In one embodiment of the invention L is absent, making the NH of the pyrrolidine ring to the FG (functional group).

### Methods

### Covalent chemical functionalization of the CNTs:

According to preferred embodiments of the present invention, functionalizations of the general formula I, of the invention can be obtained by a cycloaddition reaction between CNTs on a surface and diazometheines, formed from an amino acid or amino acid derivative and an aldehyde. The reaction can be performed in, preferably but not limited to; toluene, benzene, chlorobenzene, dichlorobenzene, poly halogenated benzene derivatives, trifluoro methylbenzene, CH₂Cl₂, CHCl₃, CCl₄, DCE, DMF, DMA, NMP, dioxane, THF, 2-MeTHF, DMSO or another suitable solvent for the reaction, at temperatures ranging from 20 - 250 °C. The reaction time can vary depending on solvent and temperature but a typically reaction time is 1 - 48 h.

Persons skilled in the art appreciate that in order to obtain a structure of the invention, one to twelve reaction steps may be needed (steps for protection and/or deprotection of protecting groups are not counted). It will be appreciated that by those skilled in the art that the process described above and hereinafter functional groups of intermediates may need to be protected by protecting groups. The type of chemistry involved will dictate the need for protecting groups as well as the sequence for accomplishing the covalent modification of CNTs on a surface. The use of protecting groups is fully described in "Protective groups in Organic Chemistry", edited by J W F McOmie, Plenum Press (1973), and "Protective Groups in Organic Synthesis", 3rd edition, T.W. Greene & P.G.M Wutz, Wiley-Interscience (1999). Protected derivates/derivatives of the invention may be converted chemically to structures of the invention using standard deprotection techniques.

### Devices

The present invention also features devices comprising a plurality of CNT structures as described herein.

In certain embodiments, the devices comprising a plurality of CNT structures host functionalizations in a high surface area 3D set up in solid, liquid, gaseous or liquid crystal or any other phases. In other embodiments, in the devices comprising a plurality of CNT structures there is energy exchange, transfer or storage between the CNT structures and the functionalizations or between the pluralities of CNT structures that are functionalized.

In other exemplary embodiments, the CNT structures as described herein host the functionalizations as electromechanical or mechanical structures.

In other further embodiments, the functionalized CNT structures are used as membranes.

In still other further embodiment, the functionalized CNT structures are used as gas storage devices

The present disclosure also features a CNT device that constitutes of one or a plurality of CNT structures grown on a surface that conducts electricity and/or heat and/or light. In other further embodiments, the disclosure features a CNT device that constitutes of one or a plurality of CNT structures grown on a surface that is an insulator of electricity and/or heat and/or light.

In certain embodiments, the CNT structures as described herein are functionalized in solid, liquid, gaseous or liquid crystal or any other phases. In further related embodiments, the CNT structures comprise a high surface area 3D set up that can be functionalized in solid, liquid, gaseous or liquid crystal or any other phases.

In other further related embodiments, the functionalized CNTs are used as sensors, energy storage devices, photovoltaic devices or imaging devices. In certain exemplary embodiments, the devices formed after functionalization of the chips can be used as sensors by providing electrical signals or thermaloelectrical or optoelectrical signals; used to store charges as batteries; used to produce or enhance the photo voltaic effect; used as imaging device to probe for materials that will be sensitive only to the functionalized species on the CNTS; contacts for molecular electronics as the molecular electronics can bind to the functional groups on CNTs and hence, charges can be then transported from the molecular electronics to the CNTs and then to conventional CMOS electronics.

### EXAMPLES

The following examples, including the experiments conducted and results achieved are provided.

### Materials and Methods

The following examples were carried out using, but not limited to, the following materials and methods.

Chemicals were obtained from commercial suppliers and used without purification unless otherwise noted. The modified CNT forest on chip was analyzed using Scanning Electron Microscopy (SEM) with Energy Dispersive Spectroscopy (EDS). The term "CNT chip" as used herein is meant to refer to carbon nanotubes grown on a silicon chip.

### Example 1: Fabrication of CNT structures

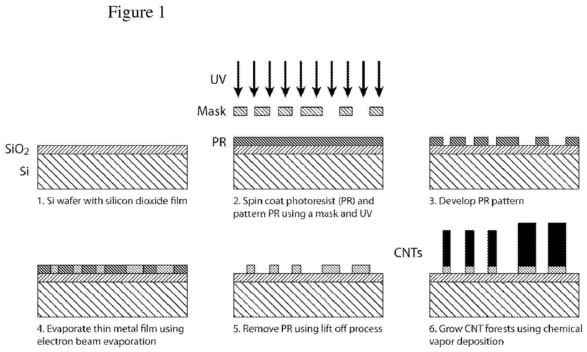

The fabrication process for the CNT forest on chip is shown in Figure 1.

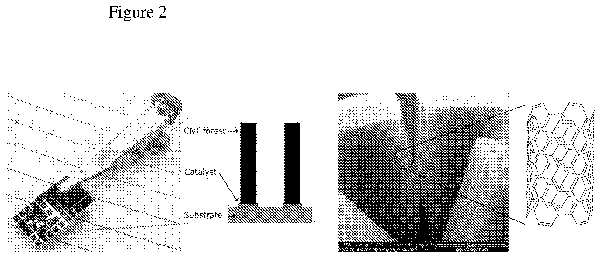

A silicon wafer with a 0.5 µm silicon oxide was used as the substrate. Using lithography, a pattern was created on a photo resist (PR) film on the substrate. Electron beam evaporation was employed to deposit thin films of metals (Ti 1nm/Mo 100 nm/Al₂O₃ 5nm/Fe 1nm). Iron film is used as a catalyst to grow CNT forests. After e-beam evaporation, the photoresist is dissolved in an organic solvent (Photoresist remover) assisted by an ultrasonic bath, a process known as lift-off. The wafer is diced into chips and CNT forests are grown on the chips using thermal chemical vapor deposition (CVD) process. In this process, the catalyst (iron) is first annealed at 500 °C for 5 minutes in the presence of Hydrogen and Nitrogen. Later, ethylene, hydrogen and nitrogen gases are added at 700 °C and hence, CNT forests are grown by CVD. The process is carried out in a cold tube furnace system (Black Magic by Aixtron). The resistance between the tips of the CNT structures range from 100 - 200 Ω. A picture and a schematic drawing of the CNT forests on chip produced are shown in Figure 2.

Another recipe used to grow them according to certain preferred embodiments of the invention is shown below in Table 1:

**Table 1**

| Step | Time | Hydrogen | Ethylene | Helium | Temp |
|---|---|---|---|---|---|
| 1 | 5 | 0 | 0 | 1000 | 25 |
| 2 | 5 | 100 | 0 | 400 | 25 |
| 3 | 10 | 100 | 0 | 400 | 775 |
| 4 | 10 | 100 | 0 | 400 | 775 |
| 5 | 30 | 100 | 100 | 400 | 775 |
| 6 | 1 | 100 | 100 | 400 | 25 |
| 7 | 15 | 100 | 100 | 400 | 25 |
| 8 | 5 | 0 | 0 | 1000 | 25 |

where time is in minutes, gases are in sccm, temperature is in Celsius. When the step temperature is different from the previous step temperature it means it is a ramp. For instance, step 3 is ramping to 775 from 25 over 10 minutes. Also, step 5 is the growth step, so the growth time is 30 minutes.

### Example 2: Functionalization Process

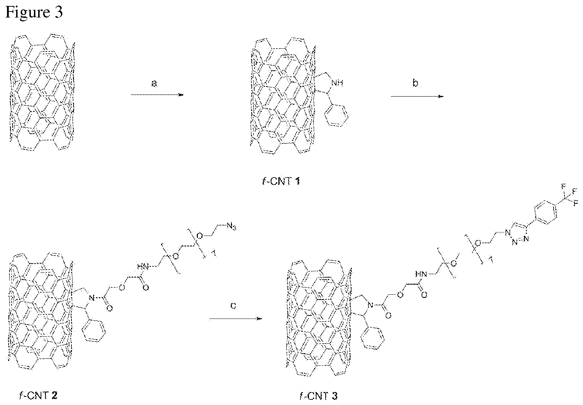

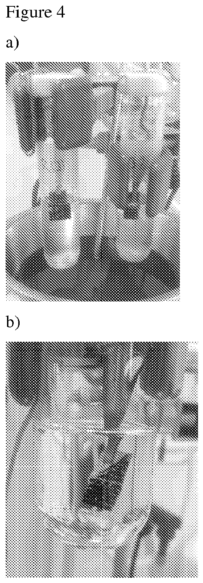

The strategy to functionalize CNT forests on chip is based on the well known 1,3-dipolar cycloaddition of azomethine ylides to C₆₀ and SWCNT in solution and is outlined in the scheme shown in Figure 3. The azomethine ylide was formed upon heating to 120 °C in dichlorobenzene from the imine formed *in situ* between glycine and benzaldehyde, and reacted with CNT forests on chip. The procedure yielded the amine-modified CNT forest on chip, *f-*CNT **1**, after cleaning. *f*-CNT **1** was further functionalized by an amide coupling with *O*-(2-azidoethyl)-*O*-[2-(diglycolyl-amino)ethyl]heptaethylene glycol to introduce azide functionalities on the CNT's. The amide coupling was carried out using PyBOP as coupling reagent under mild basic conditions (DIPEA in anhydrous CH₂Cl₂ at r.t.) to obtain azide functionalized CNT forests on chip, *f*-CNT **2.** The functionalized CNT forests on chip were analyzed using SEM with EDS. To prove that *f-*CNT **2** were functionalized with azide groups, fluorine atoms was introduced via CuAAC reaction with 1-ethynyl-4-(trifluoromethyl)benzene at r.t. to obtain *f*-CNT **3.** As a control experiment *f-*CNT **1** was also treated with 1-ethynyl-4-(trifluoromethyl)benzene under similar reaction conditions. Figure 4 shows the reaction set up and the cleaning set up.

### Experimental procedures:

*f*-CNT **1** (Cycloaddition step): In a 5 mL microwave vial were placed glycine (22 mg, 0.29 mmol), benzaldehyde (36 mg, 0.34 mmol) and anhydrous dichlorobenzene (4 mL). The mixture was ultra-sonicated for ∼30 s and the CNT chip was lowered into the solution using a copper clamp on a wire through the septum of a cap. The vial was sealed and the mixture was heated to ∼120 °C under very gentle stirring (60 rpm with a small magnetic stirring bar) for 24 h. The mixture was cooled to r.t. and the CNT chip was transferred to a new vial containing CH₂Cl₂ (15 mL) and allowed to stand for ∼1 h. This cleaning process was repeated twice and the amione modified CNT forest on chip was dried under ambient conditions and analyzed with SEM with EDS. The EDS data showed a nitrogen peak at 0.39 keV.

*f*-CNT **2** (Amide coupling step): In a 5 mL mircrowave vial were placed *O*-(2-azidoethyl)-*O*-[2-(diglycolyl-amino)ethyl]heptaethylene glycol (22 mg, 40 µmol), PyBOP (23 mg, 44 µmol) and anhydrous CH₂Cl₂ (5 mL). DIPEA (21 µL, 0.120 µmol) was added and the reaction mixture was stirred at r.t. for 1 h. The amine modified CNT forest on chip from the previous step was lowered into the solution using a copper clamp on a wire through the septum of a cap. The vial was sealed and the mixture was gently stirred (60 rpm) at r.t. for 24 h. The CNT chip was transferred to a new vial containing CH₂Cl₂ (10 mL) and allowed to stand for ∼1 h. This cleaning process was repeated twice and the azide modified CNT forest on chip was dried at ambient conditions and analyzed with SEM with EDS. The EDS data showed a nitrogen peak at 0.39 keV.

*f*-CNT **3** (CuAAC step): In a 5 mL microwave vial were placed 1-ethynyl-4-(trifluoromethyl)benzene (16.3 µL, 0.10 mmol) and DMA (3 mL). A solution of sodium ascorbate (4.5 mg, 0.023 mmol) in water (0.5 mL) was added followed by a solution of CuSO₄ (0.016 mmol) in water (0.5 mL). The mixture was stirred vigorously for 2 min and the azide modified CNT forest on chip was lowered into the solution and the reaction mixture was gently stirred (100 rpm) at r.t. for 40 h. The CNT chip was transferred to a new vial containing CH₂Cl₂ (15 mL) and allowed to stand for ∼2 h. This cleaning process was repeated twice and the triazole modified CNT forest on chip was dried at ambient conditions and analyzed with SEM with EDS. The EDS data showed a fluorine peak at 0.68 keV.

*f*-CNT **3 control step** (CuAAC control step): In a 5 mL microwave vial were placed 1-ethynyl-4-(trifluoromethyl)-benzene (16.3 µL, 0.10 mmol) and DMA (3 mL). A solution of sodium ascorbate (4.5 mg, 0.023 mmol) in water (0.5 mL) was added. The mixture was stirred vigorously for 2 min and the amine modified CNT forest on chip was lowered into the solution and the reaction mixture was gently stirred (100 rpm) at r.t. for 40h. The CNT chip was transferred to a new vial containing CH₂Cl₂ (15 mL) and allowed to stand for ∼2 h. This cleaning process was repeated twice and the unmodified CNT forest on chip was dried at ambient conditions and analyzed with SEM with EDS. The EDS data showed no fluorine peak.

*f*-CNT **4** (Amide coupling step): In a 5 mL mircrowave vial were placed 2-[2-(Fmoc-amino)ethoxy]ethoxy acetic acid (15.8 mg, 0.041 mmol), PyBOP (25.8 mg, 0.050 mmol) and anhydrous CH₂Cl₂ (5 mL). DIPEA (20 µL, 0.12 mmol) was added and the reaction mixture was stirred at r.t. for 1.5 h. The amine modified CNT forest on chip from the previous step was lowered into the solution using a copper clamp on a wire through the septum of a cap. The vial was sealed and the mixture was gently stirred (60 rpm) at r.t. for 24 h. The CNT chip was transferred to a new vial containing CH₂Cl₂ (15 mL) and allowed to stand for at least 1 h. This cleaning process was repeated twice and the Fmoc-Amine modified CNT forest on chip was dried at ambient conditions.

*f*-CNT **5** (Amide coupling step): In a 5 mL mircrowave vial were placed Fmoc-L-propargyl-Gly-OH (13.3 mg, 0.040 mmol), PyBOP (23.7 mg, 0.045 mmol) and anhydrous CH₂Cl₂ (5 mL). DIPEA (20 µL, 0.12 mmol) was added and the reaction mixture was stirred at r.t. for 1.5 h. The amine modified CNT forest on chip from the previous step was lowered into the solution using a copper clamp on a wire through the septum of a cap. The vial was sealed and the mixture was gently stirred (60 rpm) at r.t. for 24 h. The CNT chip was transferred to a new vial containing CH₂Cl₂ (15 mL) and allowed to stand for at least 1 h. This cleaning process was repeated twice and the Fmoc-Amine-Alkyne modified CNT forest on chip was dried at ambient conditions.

*f*-CNT **6** (Fmoc deprotection step): In a 5 mL mircrowave vial were placed Piperidine (0.8 mL) and DMF (3.2 mL). The Fmoc-Amine-Alkyne modified CNT forest on chip from the previous step was lowered into the solution using a copper clamp on a wire through the septum of a cap. The vial was sealed and the mixture was gently stirred (60 rpm) at r.t. for 24 h. The CNT chip was transferred to a new vial containing CH₂Cl₂ (15 mL) and allowed to stand for at least 1 h. This cleaning process was repeated twice and the Amine-Alkyne modified CNT forest on chip was dried at ambient conditions.

### Results:

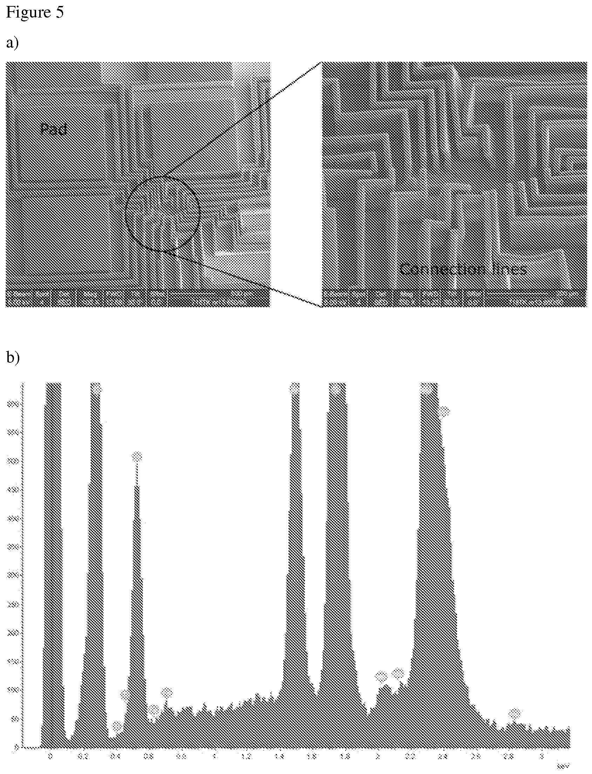

The modified CNT forests, *f*-CNT **1**, *f*-CNT **2**, *f*-CNT **3** and *f*-CNT **1** treated with reaction conditions (c) were analyzed using SEM equipped with EDS. The micrograph in Figure 5a shows the CNT structures before functionalization. The EDS spectrum in Figure 5b shows abundant elements on the chip.

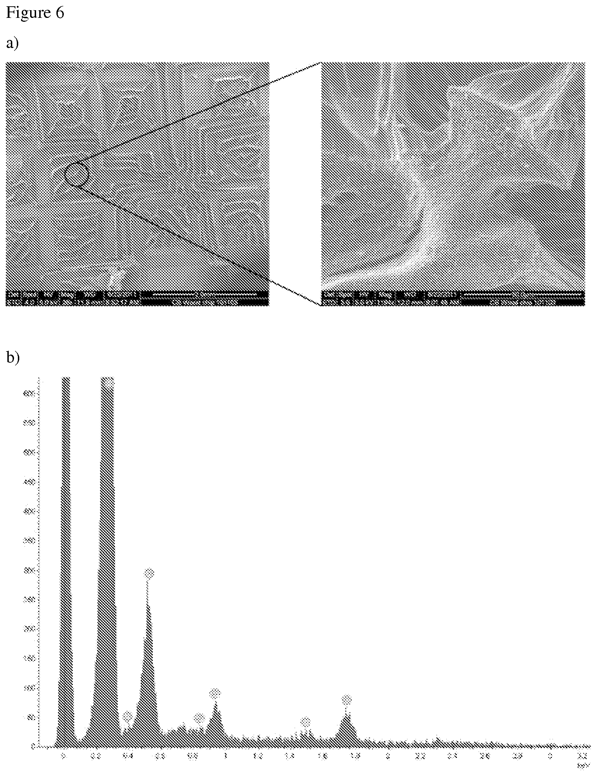

In Figure 5b peaks corresponding to carbon, silicon, oxygen, titanium, molybdenum, aluminum and iron can be seen. They are all consistent with the materials used to fabricate the chip as described in the fabrication process. The SEM micrograph of *f*-CNT **1** in Figure 6a shows the CNT structures after the cycloadditon reaction and the EDS spectrum in Figure 6b shows the elements present on the chip. Please note that the CNT forests are still maintaining the morphology of the initial structures as shown in Figure 5a.

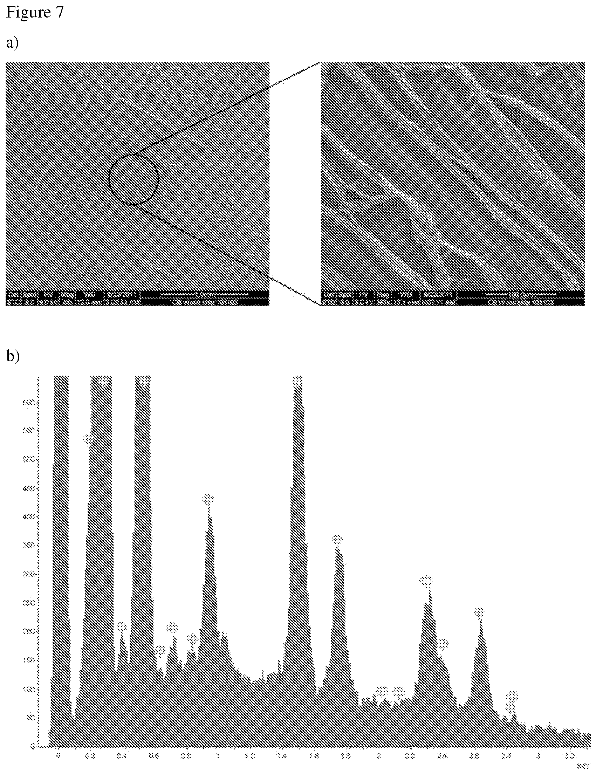

The EDS spectra in Figure 6b show a small peak of nitrogen along with the other elements also seen in the EDS spectra of an unmodified chip (Figure 5b). This indicates that the CNTs were functionalized with the cycloaddition product pyrrolidine. It is thus hypothesized that the additional Cu peak also seen in the spectra, originates from the use of a copper clamp to hold the chip during the reaction conditions (Figure 2). The same sequence of SEM and EDS was repeated with the *f*-CNT **2** chip with azide functional groups attached to the CNT structures and the results are shown in Figure 7a-b.

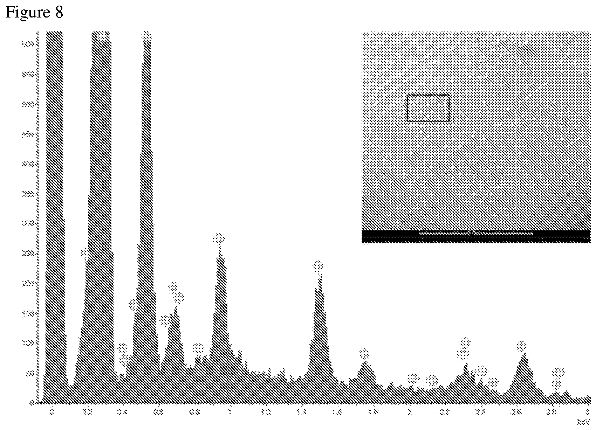

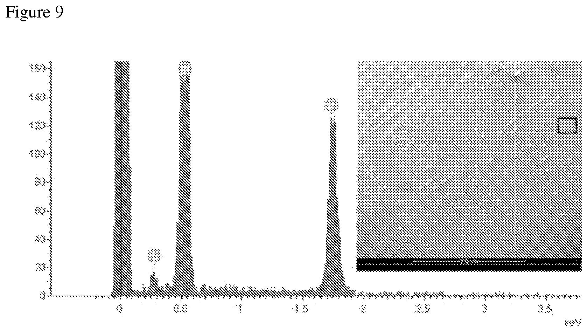

The nitrogen peak in the spectrum of *f*-CNT **2** (Figure 7b) is a little stronger than that in *f*-CNT **1** (Figure 6b) as more nitrogen is added to the CNT structures after the second reaction step. Since the difference between the two EDS spectra in Figure 6 and 7 is not large enough to give a satisfactory confirmation of the second reaction, a third reaction was performed in order to introduce a new element onto the CNT forests on-chip. A CuAAC reaction was performed to introduce fluorine that can be easily detected in EDS spectra. Figure 8 show the SEM micrograph and the EDS spectrum of *f*-CNT **3.** An EDS spectrum was also recorded in an area of the chip without any CNTs, are shown in Figure 9.

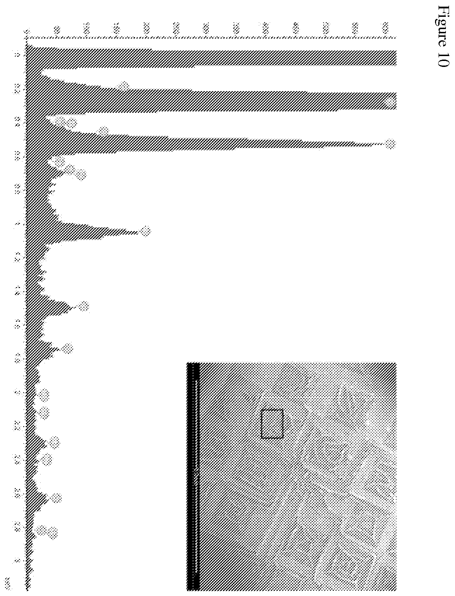

As a control that the fluorine peak in the EDS spectra does not originate from non-covalent deposition of 1-ethynyl-4-(trifluoromethyl)benzene on the CNT forests, *f-*CNT **1** was also treated under similar reaction conditions as *f-*CNT 2 above. Figure 10 shows the SEM micrograph and the EDS spectra of the control reaction.

According to the EDS spectrum in Figure 8, the fluorine peak at 0.68 keV indicates that the CNT forests are covalently functionalized with fluorine. The EDS spectrum of the control experiment (Figure 10) shows no clear fluorine peak, but only the iron peak nearby. These results together indicate that the fluorine atoms in *f-*CNT **3** are covalently attached. This also confirms that *f-*CNT **2** was successfully functionalized with active azide groups. The small peak at 0.70 keV in Figure 8 most likely originates from iron. However, this may be interpreted as some non-covalent binding of the fluorine molecules with the CNTs being present and that the fluorine peak overlaps with the iron peak nearby in the EDS spectra.

The spectrum in Figure 5b is from a CNT chip that has Ti/Mo as the metal film base (to get contact between the CNTs for conductivity) but the CNTs are not functionalized in this case. The EDS spectrum of *f*-CNT **1** (Figure 6b) and *f*-CNT **2** (Figure 7b) are obtained from similar chips after step 1 and step 2 functionalization, respectively. The nitrogen peaks in the latter spectra provide evidence that CNT structures are indeed functionalized by the reactions carried out. Interestingly, the morphology of the CNT structures is not significantly affected and the CNT structures are still electrically conducting. To further validate the *f-*CNT **2** structure, a CuAAC reaction was performed to obtain *f-*CNT **3,** which was confirmed by a fluorine peak at 0.68 keV (Figure 8). As a control reaction, *f-*CNT **1** was treated under similar conditions as *f-*CNT **2** and no fluorine peak was observed in the EDS spectra (Figure 10).

A comparison of the morphology of the CNT structures on the chips with and without functionalization of CNT forests is also presented. As seen in the SEM micrographs (Figures 6-8) the connection lines to the pads are well defined even after the functionalization of CNT structures. On the other hand, the pad structures seem somehow worn down and warped. The reason of such contrast in the morphology of CNT structures lies in the aspect ratio (the area of the catalyst on substrate. i.e. the base of the CNT forest and the length of CNTs). Since, the aspect ratio for the pads is almost a reciprocal of the aspect ratio for the contact lines, the CNTs clamp into dense sections where they can achieve the least energy structures when the solvent evaporates. The capillary forces acting on the CNTs force them to "bunch up" and form packs of dense structures. By way of contrast, the base area compared to the height of the CNTs for the contact lines is much smaller. Hence, CNTs appear to form a wall like structure, indicating that we can control the resolution of the structure (almost 5 times smaller compared to the original) once the CNT forest structures is exposed to a solvent and subsequent evaporation.

In the EDS data on *f-*CNT **2,** the presence of nitrogen indicates the successful attachment of the azide group on *f-*CNT **2** by the two-step reaction scheme described in Figure 3. However, since EDS does not provide quantitative data, a third reaction was carried out to verify the successful synthesis of the *f-*CNT **2** structure. By performing a CuAAC reaction on *f-*CNT **2** with a fluorine source, *f-*CNT **3** was successfully synthesized. The fluorine peak in the EDS spectra (Figure 8) of *f*-CNT **3** together with the absence of fluorine in the control experiment, confirms successful chemical reaction on the CNTs. This result also demonstrates that the azide functional group can be used to attach other interesting molecules to the CNT structure as they are grown on chip. Thus, the qualitative information from the EDS spectra above clearly justifies our conclusion that we have successfully achieved covalent functionalization of surface-grown CNTs on metal films as the contact metal layer.

Importantly, as concluded from the EM data, this technique does not considerably affect the morphology of the CNT forests, but the structures are preserved and, hence, may be exploited, for example, as arrays of sensing surfaces.

## Claims

1. A method of covalently functionalizing carbon nanotubes CNTs grown on a metal film surface according to formula I
SURFACE- CNT-A-L-(FG)n, comprising
a cycloaddition step;
an amide coupling step; and
a copper(I)-catalyzed azide alkyne cycloaddition (CuAAC) step,
wherein A is the attachment to the CNTs, L is a linker between the attachment on the CNTs and the one or more functional groups (FG)n, further
wherein A is a pyrrolidine ring substituted with L on either the nitrogen atom or on C2 or C5 of the pyrrolidine ring system;
wherein L is a linker between the attachment A on the CNTs and the FG, selected from the group consisting of: (C₁-C₃₀)alkyl, (C₃-C₈)cycloalkyl, aryl, heterocyclyl, and any of these groups (except hydrogen) optionally interrupted by one or more O, N, S, P, Si, or B atoms, and/or one or more carbonyls, and/or substituted by one or more halogen atoms and/or one or more of the following groups, (C₁-C₁₂)alkyl, (C₁-C₁₂)alkoxyC(O), halogen substituted (C₁-C₁₂)alkyl, (C₃-C₆)cycloalkyl, aryl, hetrocyclyl, optionally interrupted by one or more, O, N, S, P, Si or B atoms, and wherein L contains one or more carbon atoms; and
wherein the FG is selected from the group consisting of: amine, azide, alkyne, carboxylic acid, an activated ester of a carboxylic acid, thiol, acid chloride, acid fluoride, sulfonyl chloride, isocyanate, isothiocyanate, aryl halide, alkyl halide, maleimide, ketone, aldehyde, alcohol, alkene, triflate, nitro, nitrile, boronic acid, boronic ester and a trifluoroborate salt.

2. The method of claim 1, wherein A is substituted with R1 and R2, wherein R1 and R2 are each and independently selected from hydrogen, (C₁-C₁₅)alkyl, (C₃-C₈)cycloalkyl, aryl, heterocyclyl, or any of these groups (except hydrogen) optionally substituted with one or more halogen (F, Cl, Br or I) atoms and/or one or more of the following groups, OH, CN, NO₂,
(C₁-C₁₂)alkyl, (C₁-C₁₂)alkoxyC(O), halogen substituted (C₁-C₁₂)alkyl, (C₃-C₆)cycloalkyl, aryl, hetrocyclyl,
optionally interrupted by one or more, O, N, S, P, Si or B atoms.

3. The method of claim 2, wherein the C3 and C4 in A originate from the CNT structure.

4. The method of claim 2, wherein the L is bound to a nitrogen or C2 or C5 on the pyrrolidine ring system.

5. The method of claim 1, wherein the cycoladdition reaction is a 1,3-dipolar cycloaddition.

6. The method according to claim 1, wherein the method is performed in a solvent selected from the group consisting of: toluene, benzene, chlorobenzene, dichlorobenzene, poly halogenated benzene derivatives, trifluoro methylbenzene, CH₂Cl₂, CHCl₃, CCl₄, DCE, DMF, DMA, NMP, dioxane, THF, 2-MeTHF and DMSO.

7. The method of claim 1, wherein the CNTs grown on a surface are covalently functionalized on-chip.

8. A composition comprising a carbon nanotube CNT on a surface of a chip according to formula I:
SURFACE- CNT-A-L-(FG)n,
wherein A is a is a pyrrolidine ring substituted with L on either the nitrogen atom or on C2 or C5 of the pyrrolidine ring system; wherein L is a linker between the attachment A on the CNTs and the FG, selected from the group consisting of: (C₁-C₃₀)alkyl, (C₃-C₈)cycloalkyl, aryl, heterocyclyl, and any of these groups (except hydrogen) optionally interrupted by one or more O, N, S, P, Si, or B atoms, and/or one or more carbonyls, and/or substituted by one or more halogen atoms and/or one or more of the following groups, (C₁-C₁₂)alkyl, (C₁-C₁₂)alkoxyC(O), halogen substituted (C₁-C₁₂)alkyl, (C₃-C₆)cycloalkyl, aryl, hetrocyclyl, optionally interrupted by one or more, O, N, S, P, Si or B atoms, and wherein L contains one or more carbon atoms; and
wherein the (FG)n is one or more functional groups FG selected from the group consisting of:
amine, azide, alkyne, carboxylic acid, an activated ester of a carboxylic acid, thiol, acid chloride, acid fluoride, sulfonyl chloride, isocyanate, isothiocyanate, aryl halide, alkyl halide, maleimide, ketone, aldehyde, alcohol, alkene, triflate, nitro, nitrile, boronic acid, boronic ester and a trifluoroborate salt,
wherein the functional group is covalently attached,
and wherein the surface is a metal film.

9. A device comprising one or a plurality of CNT structures according to claim 8, wherein the plurality of CNT structures are covalently functionalized on-chip.

## Patentansprüche

1. Verfahren zur kovalenten Funktionalisierung von Kohlenstoffnanoröhren CNT, die auf einer Metallfilmoberfläche aufgebracht wurden, gemäß der Formel I
SURFACE- CNT-A-L-(FG)n, umfassend
einen Cycloadditionsschritt;
einen Amidkupplungsschritt; sowie
einen Kupfer(I)-katalysierten Azid-Alkin-Cycloadditions (CuAAC)-Schritt,
wobei A der Verknüpfungspunkt zu den CNT ist, L ein Linker zwischen der Verknüpfung auf den CNT und der einen oder mehreren funktionellen Gruppen (FG)n ist, und
wobei des Weiteren A ein Pyrrolidinring ist, der mit L entweder an dem Stickstoffatom oder an C2 oder C5 des Pyrrolidinringsystems substituiert ist;
worin L ein Linker zwischen der Verknüpfung A an den CNT und den FG ist, ausgewählt aus der Gruppe, bestehend aus (C₁-C₃₀)Alkyl, (C₃-C₈)Cycloalkyl, Aryl, Heterocyclyl, und einer beliebigen dieser Gruppen (mit Ausnahme von Wasserstoff), die gegebenenfalls unterbrochen ist durch ein oder mehrere O-, N-, S-, P-, Si- oder B-Atom(e), und/oder einer oder mehreren Carbonylgruppe(n), und/oder substituiert mit einem oder mehreren Halogenatom(en) und/oder einer oder mehreren der folgenden Gruppe (n) (C₁-C₁₂)Alkyl, (C₁-C₁₂)AlkoxyC(O), Halogen-substituiertem (C₁-C₁₂)Alkyl, (C₃-C₆)Cycloalkyl, Aryl, Hetrocyclyl, gegebenenfalls unterbrochen durch ein oder mehrere O-, N-, S-, P-, Si- oder B-Atom(e), und wobei L ein oder mehrere Kohlenstoffatom(e) enthält; und
worin FG ausgewählt ist aus der Gruppe, bestehend aus Amin, Azid, Alkin, Carbonsäure, einem aktivierten Ester einer Carbonsäure, Thiol, Säurechlorid, Säurefluorid, Sulfonylchlorid, Isocyanat, Isothiocyanat, Arylhalogenid, Alkylhalogenid, Maleimid, Keton, Aldehyd, Alkohol, Alken, Triflat, Nitro, Nitril, Boronsäure, Boronsäureester und einem Trifluorboratsalz.

2. Verfahren gemäß Anspruch 1, wobei A mit R1 und R2 substituiert ist, wobei R1 und R2 jeweils und unabhängig ausgewählt sind aus Wasserstoff, (C₁-C₁₅)Alkyl, (C₃-C₈)Cycloalkyl, Aryl, Heterocyclyl, oder einer beliebigen dieser Gruppen (mit Ausnahme von Wasserstoff), gegebenenfalls substituiert mit einem oder mehreren Halogenatomen (F, Cl, Br oder I), und/oder einer oder mehreren der folgenden Gruppen: OH, CN, NO₂, (C₁-C₁₂)Alkyl, (C₁-C₁₂)AlkoxyC(O), Halogen-substituiertem (C₁-C₁₂)Alkyl, (C₃-C₆)Cycloalkyl, Aryl, Hetrocyclyl, gegebenenfalls unterbrochen durch eine oder mehrere O-, N-, S-, P-, Si- oder B-Atome.

3. Verfahren gemäß Anspruch 2, wobei C3 und C4 in A aus der CNT-Struktur herrühren.

4. Verfahren gemäß Anspruch 2, wobei L an ein Stickstoffatom oder C2 oder C5 am Pyrrolidinringsystem gebunden ist.

5. Verfahren gemäß Anspruch 1, wobei die Cycloadditionsreaktion eine 1,3-dipolare Cycloaddition ist.

6. Verfahren gemäß Anspruch 1, wobei das Verfahren in einem Lösungsmittel ausgeführt wird, ausgewählt aus der Gruppe, bestehend aus Toluol, Benzol, Chlorbenzol, Dichlorbenzol, polyhalogenierten Benzolderivaten, Trifluormethylbenzol, CH₂Cl₂, CHCl₃, CCl₄, DCE, DMF, DMA, NMP, Dioxan, THF, 2-MeTHF und DMSO.

7. Verfahren gemäß Anspruch 1, wobei die auf einer Oberfläche aufgebrachten CNT kovalent On-Chip-funktionalisiert sind.

8. Zusammensetzung, umfassend eine Kohlenstoffnanoröhre CNT auf einer Oberfläche eines Chips gemäß Formel I:
SURFACE- CNT-A-L-(FG)n,
wobei A ein Pyrrolidinring ist, der mit L entweder an dem Stickstoffatom oder an C2 oder C5 des Pyrrolidinringsystems substituiert ist;
worin L ein Linker zwischen der Verknüpfung A an den CNT und den FG ist, ausgewählt aus der Gruppe, bestehend aus (C₁-C₃₀)Alkyl, (C₃-C₈)Cycloalkyl, Aryl, Heterocyclyl, und einer beliebigen dieser Gruppen (mit Ausnahme von Wasserstoff), die gegebenenfalls unterbrochen ist durch ein oder mehrere O-, N-, S-, P-, Si- oder B-Atom(e), und/oder einer oder mehreren Carbonylgruppe(n), und/oder substituiert mit einem oder mehreren Halogenatom(en) und/oder einer oder mehreren der folgenden Gruppe (n) (C₁-C₁₂)Alkyl, (C₁-C₁₂)AlkoxyC(O), Halogen-substituiertem (C₁-C₁₂)Alkyl, (C₃-C₆)Cycloalkyl, Aryl, Hetrocyclyl, gegebenenfalls unterbrochen durch ein oder mehrere O-, N-, S-, P-, Si- oder B-Atom(e), und wobei L ein oder mehrere Kohlenstoffatom(e) enthält; und
worin FG ausgewählt ist aus der Gruppe, bestehend aus Amin, Azid, Alkin, Carbonsäure, einem aktivierten Ester einer Carbonsäure, Thiol, Säurechlorid, Säurefluorid, Sulfonylchlorid, Isocyanat, Isothiocyanat, Arylhalogenid, Alkylhalogenid, Maleimid, Keton, Aldehyd, Alkohol, Alken, Triflat, Nitro, Nitril, Boronsäure, Boronsäureester und einem Trifluorboratsalz, wobei die funktionelle Gruppe kovalent verknüpft ist, und wobei die Oberfläche ein Metallfilm ist.

9. Vorrichtung, umfassend eine oder eine Mehrzahl CNT-Strukturen gemäß Anspruch 8, wobei die Mehrzahl CNT-Strukturen kovalent On-Chip-funktionalisiert ist.

## Revendications

1. Procédé de fonctionnalisation covalente de nanotubes de carbone NTC amenés à croître sur une surface de film métallique selon la formule I
SURFACE-CNT-A-L-(FG)n,
comprenant
une étape de cyclo-addition;
une étape de couplage par liaison amide; et
une étape de cyclo-addition d'azoture et d'alcyne catalysée au cuivre (I) (CuAAC),
où A est la fixation aux CNT, L est un agent de liaison entre la fixation sur les CNT et les un ou plusieurs groupes fonctionnels (FG)n, par ailleurs
où A est un noyau de pyrrolidine substitué par L soit sur l'atome d'azote, soit sur C2 ou C5 du système de noyau de pyrrolidine;
où L est un agent de liaison entre la fixation A sur les CNT et le FG, choisi dans le groupe constitué de: (C₁-C₃₀)alkyle, (C₃-C₈)cycloalkyle, aryle, hétérocyclyle, et l'un ou l'autre de ces groupes (à l'exception de l'hydrogène) optionnellement interrompu par un ou plusieurs atomes de O, N, S, P, Si ou B, et/ou un ou plusieurs carbonyles, et/ou substitué par un ou plusieurs atomes d'halogène et/ou un ou plusieurs des groupes suivants, (C₁-C₁₂)alkyle, (C₁-C₁₂)alcoxyC(O), (C₁-C₁₂)alkyle substitué par un halogène, (C₃-C₆)cycloalkyle, aryle, hétérocyclyle, optionnellement interrompu par un ou plusieurs atomes de O, N, S, P, Si ou B, et où L contient un ou plusieurs atomes de carbone; et
où le FG est choisi dans le groupe constitué de: amine, azoture, alcyne, acide carboxylique, un ester activé d'un acide carboxylique, thiol, chlorure d'acide, fluorure d'acide, chlorure de sulfonyle, isocyanate, isothiocyanate, halogénure d'aryle, halogénure d'alkyle, maléimide, cétone, aldéhyde, alcool, alcène, triflate, nitro, nitrile, acide boronique, ester boronique et un sel de trifluoroborate.

2. Procédé selon la revendication 1, dans lequel A est substitué par R1 et R2, où R1 et R2 sont, chacun, choisis indépendamment parmi hydrogène, (C₁-C₁₅)alkyle, (C₃-C₈)cycloalkyle, aryle, hétérocyclyle, ou l'un ou l'autre de ces groupes (à l'exception de l'hydrogène) optionnellement substitué par un ou plusieurs atomes d'halogène (F, CI, Br ou I) et/ou un ou plusieurs des groupes suivants, OH, CN, NO₂, (C₁-C₁₂)alkyle, (C₁-C₁₂)alcoxyC(O), (C₁-C₁₂)alkyle substitué par un halogène, (C₃-C₆)cycloalkyle, aryle, hétérocyclyle, optionnellement interrompu par un ou plusieurs atomes de O, N, S, P, Si ou B.

3. Procédé selon la revendication 2, dans lequel les C3 et C4 dans A proviennent de la structure de CNT.

4. Procédé selon la revendication 2, dans lequel le L est lié à un azote ou C2 ou C5 sur le système de noyau de pyrrolidine.

5. Procédé selon la revendication 1, dans lequel la réaction de cyclo-addition est une cyclo-addition 1,3-dipolaire.

6. Procédé selon l'une quelconque des revendications précédentes, **caractérisé par le fait qu'**il est effectué dans un solvant choisi dans le groupe constitué par: toluène, benzène, chlorobenzène, dichlorobenzène, les dérivés poly-halogénés de benzène, trifluoro-méthylbenzène, CH₂CI₃, CHCI₃, CCI₄, DCE, DMF, DMA, NMP, dioxane, THF, 2-MeTHF et DMSO.

7. Procédé selon la revendication 1, dans lequel les NTC amenés à croître sur une surface sont fonctionnalisés de manière covalente sur une puce.

8. Composition comprenant un nanotube de carbone CNT sur une surface d'une puce selon la formule I:
SURFACE- CNT-A-L-(FG)n,
où A est un noyau de pyrrolidine substitué par L soit sur l'atome d'azote, soit sur C2 ou C5 du système de noyau de pyrrolidine;
où L est un agent de liaison entre la fixation A sur les CNT et le FG, choisi dans le groupe constitué de: (C₁-C₃₀)alkyle, (C₃-C₈)cycloalkyle, aryle, hétérocyclyle, et l'un ou l'autre de ces groupes (à l'exception de l'hydrogène) optionnellement interrompu par un ou plusieurs atomes de O, N, S, P, Si ou B, et/ou un ou plusieurs carbonyles, et/ou substitué par un ou plusieurs atomes d'halogène et/ou un ou plusieurs des groupes suivants, (C₁-C₁₂)alkyle, (C₁-C₁₂)alcoxyC(O), (C₁-C₁₂)alkyle substitué par un halogène, (C₃-C₆)cycloalkyle, aryle, hétérocyclyle, optionnellement interrompu par un ou plusieurs atomes de O, N, S, P, Si ou B, et où L contient un ou plusieurs atomes de carbone; et
où le FG est un ou plusieurs groupes fonctionnels FG choisis dans le groupe constitué de: amine, azoture, alcyne, acide carboxylique, un ester activé d'un acide carboxylique, thiol, chlorure d'acide, fluorure d'acide, chlorure de sulfonyle, isocyanate, isothiocyanate, halogénure d'aryle, halogénure d'alkyle, maléimide, cétone, aldéhyde, alcool, alcène, triflate, nitro, nitrile, acide boronique, ester boronique et un sel de trifluoroborate,
dans lequel le groupe fonctionnel est fixé de manière covalente,
et dans lequel la surface est un film métallique.

9. Dispositif comprenant une ou une pluralité de structures de NTC selon la revendication 8, **caractérisé par le fait que** la pluralité de structures de NTC sont fonctionnalisées de manière covalente sur une puce.
